Europäisches Patentamt

European Patent Office

Office européen des brevets

(19)

(11) Publication number: **0 181 067 B1**

# EUROPEAN PATENT SPECIFICATION

(45) Date of publication of patent specification: **31.07.91**

(51) Int. Cl.⁵: **A61B 5/02**, A61B 5/0245, A61B 5/0285

(21) Application number: 85306390.7

(22) Date of filing: **09.09.85**

(54) Device for displaying a blood pressure value.

(30) Priority: **10.09.84 GB 8422770**

(43) Date of publication of application:
**14.05.86 Bulletin 86/20**

(45) Publication of the grant of the patent:
**31.07.91 Bulletin 91/31**

(84) Designated Contracting States:
**AT BE CH DE FR GB IT LI NL SE**

(56) References cited:
EP-A- 0 021 800
DE-B- 2 537 485
US-A- 4 245 648

(73) Proprietor: **PULSE TIME PRODUCTS LIMITED
Sussex Suite City Gates
Southgate Chichester West Sussex, PO19
2DJ(GB)**

(72) Inventor: **Orr, Thomas
Flat 4, Dolphin Court Marine Parade West
Lee-on-the Solent Hampshire PO13 9LW(GB)**
Inventor: **Carruthers, Malcolm E.
Positive Health Ltd. 15 Fitzroy Square
London W1P 5HQ(GB)**

(74) Representative: **Moon, Donald Keith et al
BREWER & SON Quality House Quality Court
Chancery Lane
London WC2A 1HT(GB)**

**Description**

The present invention relates to a method of, and device for, displaying a value or index of blood pressure within a living human or animal body which method includes the steps of sensing the emission of electrocardiographic R-waves, sensing the arrival of consequent pulses of blood at a chosen blood vessel and computing i) an elapsed time TT between the sensed instant of emission of each R-wave and the sensed instant of arrival of the consequent pulse, and ii) a heart beat rate HR, being a sensed number of R-waves occurring in a given unit of time.

Such a method is described in European Patent Application No. EP-A-0021800, which also discloses apparatus for carrying out such a method, but the disclosure concerns only the monitoring and display of a generalised blood pressure, not indicative of an underlying diastolic blood pressure. Strenuous exercise has a general effect of raising systolic blood pressure and heart rate, but has less effect on diastolic blood pressure.

In the field of preventative medical treatment of heart disease, there has long been a necessity for routine long term monitoring of diastolic blood pressure without significant disturbance of the ordinary life of the subject.

It is one object of the present invention to provide a method of indicating in real time at least one of i) changes in, and ii) absolute values of, diastolic blood pressure, using data obtained from personal portable sensing apparatus.

According to a first aspect of the present invention there is provided a method of displaying a value or index of blood pressure within a living human or animal body which includes the step of sensing the emission of the electrocardiographic R-waves, sensing the arrival of consequent pulses of blood at a chosen blood vessel and computing i) an elapsed time TT between the sensed instant of emission of each R-wave and the sensed instant of arrival of the consequent pressure pulse, and ii) a heart beat rate HR, being a sensed number of R-waves occurring in a given unit of time, and characterised by the steps of: a) finding the product of HR and TT; b) dividing this product ( TT.HR ) by a heart beat rate, $HR_c$, measured when the subject is at rest, to obtain a parameter $TT.HR / HR_c$, c) computing a value or index of diastolic blood pressure using function, F1, quoted below:-

$$DBP = m \cdot TT \cdot \frac{HR}{HR_C} + I \quad \ldots\ldots\ldots\ldots\ldots \quad F1$$

wherein

m is the gradient of best fit of a straight line plot of the variation of absolute diastolic blood pressure (ordinate) against

$$TT \cdot \frac{HR}{HR_C}$$

(abscissa):

$HA_c$ is an at rest heart beat rate:

I is the intercept of the said straight line plot with the ordinate;

and m, $HR_c$, and I are numerical constants determined during the calibration procedure, and d) displaying the said value or index of diastolic blood pressure.

According to a second aspect of the present invention, there is provided a device for displaying a value or index of blood pressure within a living human or animal body which includes means for sensing the emission of the electrocardiographic R-waves, means for sensing the arrival of consequent pulses of blood at a chosen blood vessel and means for computing i) an elapsed time TT between the sensed instant of emission of each R-wave and the sensed instant of arrival of the consequent pressure pulse, and ii) a heart beat rate HR, being a sensed number of R-waves occurring in a given unit of time, and characterised by computing means (24) for: a) finding the product of HR and TT; b) dividing this product ( TT.HR ) by a heart beat rate, $HR_c$, measured when the subject is at rest, to obtain a parameter $TT.HR / HR_c$, c) computing a value or index of diastolic blood pressure using function, F1, quoted below:-

$$DBP \quad = m \; . \; TT \; . \; \frac{HR}{HR_C} \; + \; I \qquad \ldots\ldots\ldots\ldots\ldots \qquad F1$$

wherein

m is the gradient of best fit of a straight line plot of the variation of absolute diastolic blood pressure (ordinate) against

$$TT \; . \; \frac{HR}{HR_C}$$

(abscissa):

$HR_c$ is an at rest heart beat rate:

I is the intercept of the said straight line plot with the ordinate;

and m, $HR_c$, and I are numerical constants determined during the calibration procedure, and d) displaying the said value or index of diastolic blood pressure.

Underlying the present invention is an appreciation by the applicants, deriving from their own experiments and observations, that the diastolic blood pressure of any particular individual remains substantially unchanged irrespective of the degree of exercise or stress to which the body is subject, unlike the systolic pressure which tends to increase in proportion to the degree of exercise. It will be appreciated that, under stress, HR increases but TT falls. Thus, the product HR.TT changes less than either TT or HR.

The function on which the computing step is performed is F1 below:

$$DBP \; = \; m.TT.\frac{HR}{HR_C} \; + \; I \; \ldots\ldots\ldots\ldots\ldots \; F1$$

wherein m, $HR_c$ and I are all numerical constants and, more particularly:

m is the gradient of best fit of a straight line plot of the variation of absolute diastolic blood pressure (ordinate) against

$$\frac{TT.HR}{HRc}$$

(abscissa), m usually having a value of around - 0.06 mmHg/ms and TT being usually in a range of from 100 to 300 ms;

I is the notional intercept of the straight line of gradient m with the said ordinate, the intercept usually having a value of some tens of millimetres of mercury;

$HR_c$ is a constant which represents an at rest heart beat rate; and

DBP is a displayed value of diastolic blood pressure, conveniently expressed in units of mm.Hg, normally of a value a little below 100.

The value of DBP changes relatively little with stress or exercise. The experiments which the applicants have conducted have provided empirical evidence of the truth of the formula F1.

The calibration constants m and I can be determined, for any particular subject, by measurements on that subject using an absolute diastolic blood pressure meter i.e. a sphygmomanometer. The calibration constant $HR_c$ can be determined using any convenient method of establishing an at-rest heart beat rate.

Real time measurements of current heart beat rate HR can be determined in the way described in the European patent application mentioned above, or in any other convenient manner.

Elapsed time TT again can be measured as described in the European patent application. It is, however, not entirely straightforward to obtain an accurate measurement of TT, because of the relatively slow rate of rise of blood pressure in the blood vessel upon arrival of the blood pressure pulse. One convenient way of determining the moment of arrival of the pulse is to establish the pressure p of the peak of the pulse, the minimum pressure q in the trough immediately preceding the pulse, and then to establish what is the time of arrival in the blood vessel of a blood pressure which is mid-way between the measured peak pressure and the trough pressure. Because the rate of change of pressure in the pulse at this point in the pressure curve is relatively fast, any given inaccuracy in measurement of the peak and trough pressures will give rise to only a very small inaccuracy in the calculated time of arrival of the median pressure.

Apparatus in accordance with the invention, which is preferably personal and portable, has means to sense cardiographic R-waves and pulses of blood, and means to compute the quantities TT and HR as defined above, and preferred embodiments are characterised by means to compute DBP as defined above, from TT and HR, in accordance with formula F1. Changes of diastolic blood pressure from an "at rest" origin can be displayed graphically on the basis of measured values of TT, HR and $HR_c$ by using an assumed value of m. Absolute values can be displayed if the device is calibrated at two points on the straight line plot of F1, thereby to fix m and I for the specific subject under test.

In one embodiment, the apparatus might be in the form of a device worn on the wrist, and in another it is a small hand-held device. It is convenient for the step of sensing the arrival of the blood pressure pulse to be conducted in relation to a blood vessel in a thumb or fingertip of the subject.

It is convenient to provide the apparatus with means to display not only one or both of an indication of changes in diastolic blood pressure and an absolute diastolic blood pressure, but also a numerical indication of systolic blood pressure and/or heart beat rate. Preferably, the device provides a display of time or of further functions, such as those now provided as a matter of routine in microprocessor-based timepiece devices, so that it can display heart performance recovery data following, for example, a specified exercise programme.

Reference will now be made, by way of example, to the accompanying drawings, in which:

Figure 1 shows schematically the form of electrical and blood pressure pulses characteristic of a beating heart;

Figure 2 is a block diagram of components of a device in accordance with the present invention;

Figure 3 is a block diagram of the electrical pulse sensor of a preferred embodiment of the invention;

Figure 4 is a block diagram of the pressure pulse sensor of the said embodiment;

Figure 5 is a graph of function F1; and

Figure 6 is a perspective view of the said embodiment.

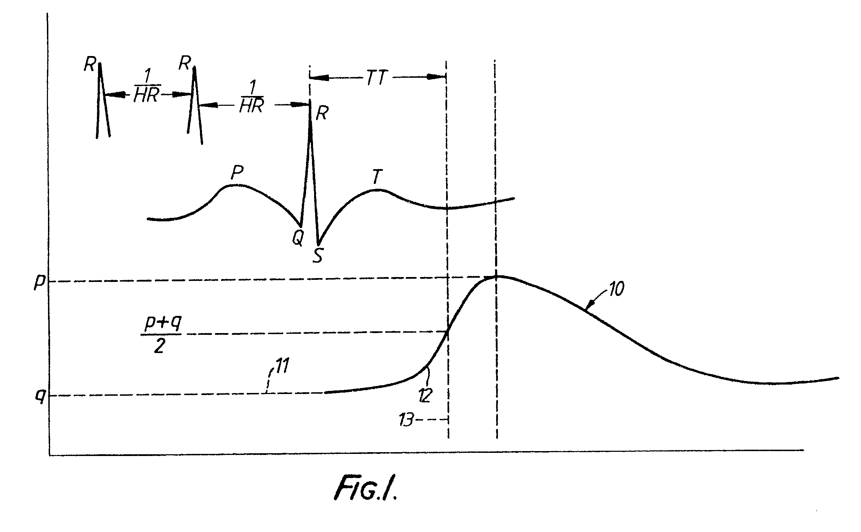

Referring to Figure 1, each electrical pulse (R-wave) is identified, as is conventional, with the letters PQRST wherein R identifies the sharp peak of the pulse. The frequency of such pulses is HR. The abscissa of the Figure 1 graph represents time, so the horizontal distance between successive peaks R is 1/HR.

The blood pressure pulse 10 shows a period 11 of steady pressure q ahead of the pulse, a period 12 of rapidly rising pressure and a peak pressure p. One convenient way of determining the instant 13 of arrival of the pulse 10 is to define it as the instant when the pressure is (p + q)/2. As can be seen from Figure 1, TT is the delay from the instant of the electrical peak R to the consequent instant 13 of arrival of the pressure pulse 10 at the blood vessel where the pressure is being sensed.

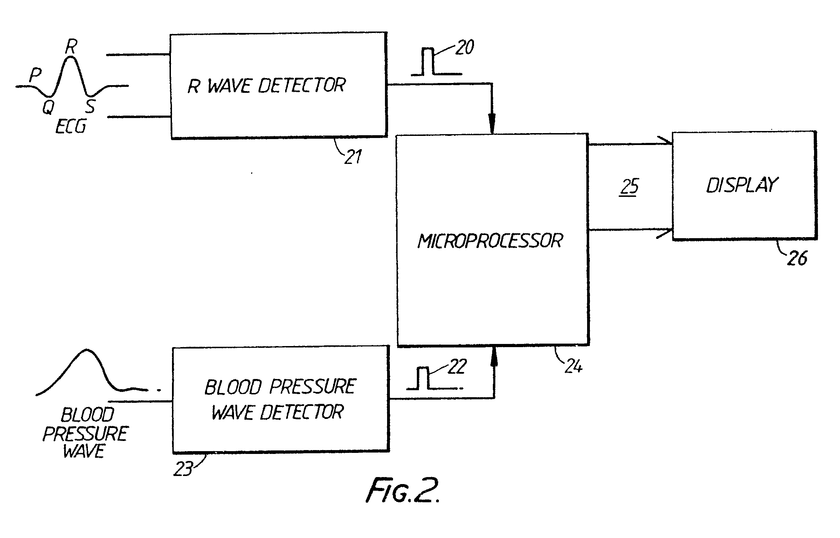

Figure 2 shows how a signal 20 from an R-wave sensor 21, and a signal 22 from a pressure sensor 23, are inputted to a microprocessor 24, the output 25 of which is delivered to a display means 26.

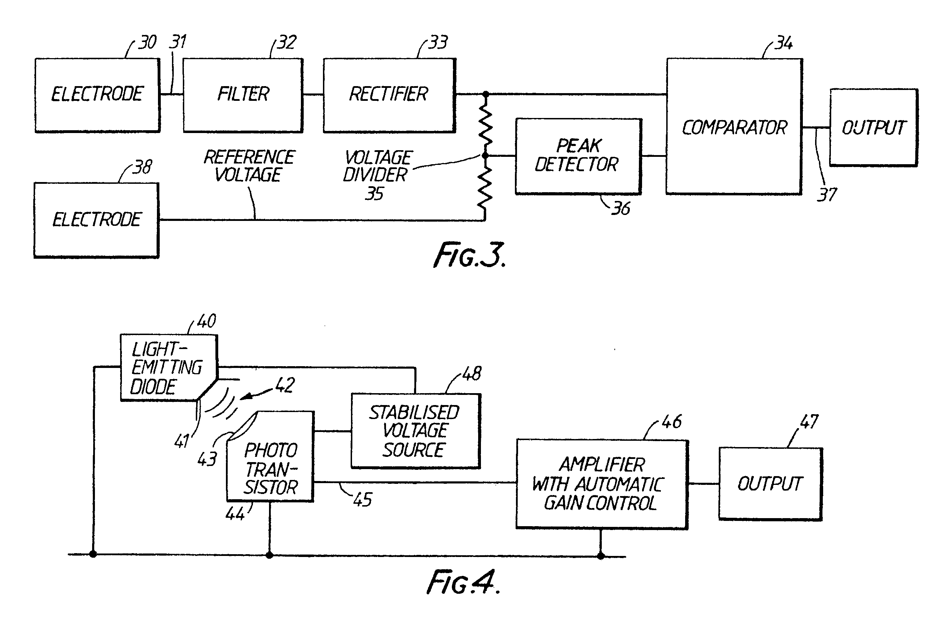

Figure 3 shows the R-wave sensor 21 in greater detail. A first skin-contact electrode 30 outputs a signal 31 through a three stage band-pass filter 32 and a full-wave rectifier 33 to a comparator 34 and a voltage divider 35. The divider 35 inputs a peak detector 36 which provides a second input to the comparator 34, which generates an output signal 37 whenever the instantaneous signal from the electrode 30 exceeds 0.83 of the magnitude of the average signal from the electrode. A reference electrode 38 provides a reference voltage to the divider 35.

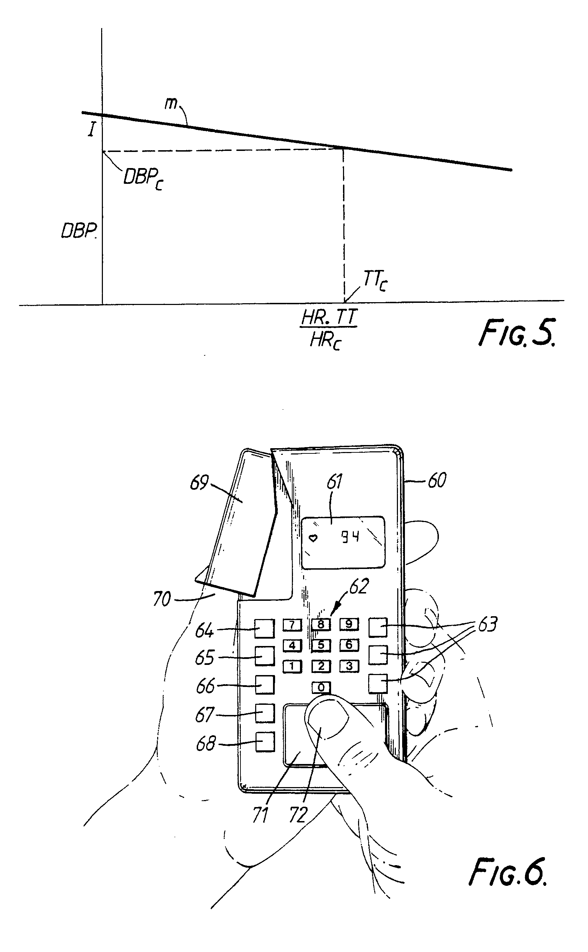

Figure 4 shows how a light-emitting diode 40 provides illumination 41 to an area 42 of skin of the human body being monitored, the intensity of consequent illumination 43 of an adjacent phototransistor 44 varying with pressure of blood in vessels immediately below the area 42 of skin. The output 45 from the transistor is delivered to an amplifier 46 with automatic gain control, and its output 47 inputs the microprocessor 24. The diode 40, transistor 44 and amplifier 46 are all powered from a stabilised voltage source 48.

Figure 5 shows a graph showing the linear variation of DBP with the quantity $(HR.TT)/HR_c$. The gradient m is usually negative and of the order of - 0.06. By measuring an "at rest" heart beat rate $HR_c$, and an elapsed time TTc at that instant, one can display an "index" of diastolic blood pressure with the origin DBPc of the index (conveniently displayed as the origin of a bar graph) at the moment of setting the "at rest" rate, and an assumed gradient m of, say, - 0.06 mmHg/ms. Otherwise, one can calibrate the device, to find a real gradient m and intercept I, so allowing measured values of DBP to be displayed.

Figure 6 shows the device in use. The circuits of Figures 2 to 4 are contained within a casing 60 in which is a display panel 61. A conventional numeric key pad 62 and chronometer keys 63 are provided.

The functions of the five other keys, namely, ENTER 64, SET 65, AUDIO/SPLIT 66, MODE 67 and CAL 68 are briefly described below. Within a cuff 69 is a sensing element comprising an LED 40 and phototransistor 44 arrangement, as shown schematically in Figure 4, for sensing the arrival of pulses of blood pressure in the tip of a digit 70 pressed against the sensing element within the cuff 69. A conductive area 71 on the front of the casing 60 is used to sense ECG R-waves in another digit 72 of the subject.

In use, the MODE key 67 is used to select one of the following modes: CHRONO, PRESSURE INDEX, PRESSURE VALUE. Use in the CHRONO mode is conventional and so will not be described.

In PRESSURE INDEX (PI) mode, pulse rate HR in beats/minute is displayed in the panel 61, as well as a bar graph showing the magnitude of both systolic and diastolic blood pressures relative to "at rest" values. To input the microprocessor with new "at rest" values, the CAL key 68 is depressed during monitoring. Relative values are computed and displayed on the basis of an assumed value of gradient m.

The device has an audio output (not shown) which can be actuated by depressing the AUDIO key 66 to signal each heart beat. Furthermore, the SET key 65 can be used to input numerical heart rate minimum and maximum values at which the audio output will sound an alarm. Using this facility an exercise programme can be pursued in which heart rate is maintained within a specified band of elevated heart rates. The function is continued so long as the conductive area 71 receives ECG signals. A digit 70 in the cuff 69 is not needed. By invoking the CNRONO mode a heart beat recovery time can be determined.

In PRESSURE VALUE (PV) mode, the device requires calibration. For this, the actual heart rate is noted and then, in PV mode, the CAL key 68 is depressed and an "at rest" numerical reference display value noted. Simultaneously, a sphygmomanometer is used to measure absolute values of diastolic and systolic blood pressure. The calibration procedure is repeated immediately following body exercise, to obtain equivalent values at a higher heart rate.

Once these values are established they are inputted to the microprocessor by depressing the SET key 65 and depressing numeral keys as prompted by the display 61. As in the PI mode, specified limit values can be entered which, when reached, cause an audible warning to be emitted.

## Claims

1. A method of displaying a value or index of blood pressure within a living human or animal body which includes the step of sensing the emission of the electrocardiographic R-waves, sensing the arrival of consequent pulses of blood at a chosen blood vessel and computing i) an elapsed time TT between the sensed instant of emission of each R-wave and the sensed instant of arrival of the consequent pressure pulse, and ii) a heart beat rate HR, being a sensed number of R-waves occurring in a given unit of time, and characterised by the steps of: a) finding the product of HR and TT; b) dividing this product ( TT.HR ) by a heart beat rate, $HR_c$, measured when the subject is at rest, to obtain a parameter $TT.HR / HR_c$, c) computing a value or index of diastolic blood pressure using function, F1, quoted below:-

$$DBP = m \cdot TT \cdot \frac{HR}{HR_c} + I \quad \ldots\ldots\ldots\ldots\ldots\ldots \quad F1$$

wherein

m is the gradient of best fit of a straight line plot of the variation of absolute diastolic blood pressure (ordinate) against

$$TT \cdot \frac{HR}{HR_c}$$

(abscissa):

$HR_c$ is an at rest heart beat rate:

I is the intercept of the said straight line plot with the ordinate;

and m, $HR_c$, and I are numerical constants determined during the calibration procedure, and d) displaying the said value or index of diastolic blood pressure.

2. A method according to claim 1 characterised by the step of performing a calibration procedure for the particular body for which the diastolic blood pressure value is to be displayed, the procedure involving the conventional measurement of absolute diastolic blood pressures, heart beat rates and elapsed times TT to determine the constants m, $HR_c$ and I for the body in question.

3. A method according to claim 1 characterised by the steps of establishing $HR_c$ and a corresponding value of TT for the particular body for which the diastolic blood pressure value is to be displayed, and performing said computing step on the basis of an assumed value of m and the actual measured values of HR and TT.

4. A method according to any one of the preceding claims characterised by defining the instant of arrival of the pulse as the instant when the increasing measured blood pressure attains a magnitude which is midway between the peak pressure of the pulse and the trough pressure which precedes the arrival of the pulse.

5. A device for displaying a value or index of blood pressure within a living human or animal body which includes means for sensing the emission of the electrocardiographic R-waves, means for sensing the arrival of consequent pulses of blood at a chosen blood vessel, and means for computing i) an elapsed time TT between the sensed instant of emission of each R-wave and the sensed instant of arrival of the consequent pressure pulse, and ii) a heart beat rate HR, being a sensed number of R-waves occurring in a given unit of time, and characterised by computing means (24) for: a) finding the product of HR and TT; b) dividing this product ( TT.HR ) by a heart beat rate, $HR_c$, measured when the subject is at rest, to obtain a parameter TT.HR / $HR_c$, c) computing a value or index of diastolic blood pressure using function, F1, quoted below:-

$$DBP \;=\; m \,.\, TT \,.\, \frac{HR}{HR_c} \;+\; I \qquad \dots\dots\dots\dots\dots\dots \qquad F1$$

wherein

m is the gradient of best fit of a straight line plot of the variation of absolute diastolic blood pressure (ordinate) against

$$TT \,.\, \frac{HR}{HR_c}$$

(abscissa):

$HR_c$ is an at rest heart beat rate:

I is the intercept of the said straight line plot with the ordinate;

and m, $HR_c$, and I are numerical constants determined during the calibration procedure, and d) displaying the said value or index of diastolic blood pressure.

6. A device as claimed in claim 5 characterised in that the computing means (24) is programmed to output an index of diastolic pressure based on an assumed value of m and an actual value of $HR_c$ which is specific to the body in question.

7. A device as claimed in claim 5 or 6 characterised in that the computing means is programmed to compute actual values of m and I which are specific to the body in question, on the basis of F1 and inputted conventionally obtained real values of diastolic pressure.

8. A device as claimed in any one of claims 5 to 7 characterised in that the computing means is programmed to determine the end of the elapsed time TT as the instant when the increasing blood pressure which marks the arrival of a pulse attains a value

6

$$\frac{p + q}{2}$$

midway (13) between the steady pressure q which precedes the pulse (10) and the peak pressure q attained during the pulse.

9. A device as claimed in any one of claims 5 to 8 characterised in that it is adapted to detect pressure pulses in blood vessels in the finger tip (70) of a human body.

10. A device as claimed in any one of claims 5 to 9 characterised in that it provides a chronometer mode which can be used in conjunction with its heart monitoring function.

**Revendications**

1. Procédé d'affichage d'une valeur ou d'un indice de pression sanguine dans un corps vivant humain ou animal, qui comprend les étapes suivantes : la détection de l'émission des ondes électrocardiographiques R, la détection de l'arrivée des impulsions sanguines correspondantes dans un vaisseau sanguin choisi, et le calcul i) d'un temps TT qui s'écoule entre le moment détecté de l'émission de chaque onde R et le moment détecté d'arrivée de l'impulsion correspondante de pression, et ii) d'un rythme cardiaque HR qui est le nombre détecté d'ondes R apparaissant pendant une unité déterminée de temps, et caractérisé par les étapes suivantes : a) la détermination du produit de HR et TT, b) la division du produit (TT.HR) par un rythme cardiaque $HR_c$ mesuré lorsque le sujet est au repos afin qu'un paramètre $TT.HR/HR_c$ soit obtenu, c) le calcul d'une valeur ou d'un indice de pression sanguine diastolique à l'aide de la fonction suivante F1 :

$$DBP = m.TT.(HR/HR_c) + I \qquad F1$$

dans laquelle :

m est la pente de la droite représentant le mieux la variation de la pression sanguine diastolique absolue (portée en ordonnées) en fonction de $TT.HR/HR_c$ (porté en abscisses),

$HR_c$ est un rythme cardiaque au repos,

I est l'intersection de la droite avec l'axe des ordonnées, et

m, $HR_c$ et I sont des constantes numériques déterminées dans la procédure d'étalonnage, et d) l'affichage de la valeur ou de l'indice de pression sanguine diastolique.

2. Procédé selon la revendication 1, caractérisé par une étape d'exécution d'une procédure d'étalonnage pour le corps particulier pour lequel la valeur de la pression sanguine diastolique doit être affichée, cette procédure comprenant la mesure classique des pressions sanguines diastoliques absolues, des rythmes cardiaques et des temps écoulés TT afin que les constantes m, $HR_c$ et I soient déterminées pour le corps considéré.

3. Procédé selon la revendication 1, caractérisé par des étapes d'établissement de $HR_c$ et d'une valeur correspondante de TT pour le corps particulier dont la valeur de la pression sanguine diastolique doit être affichée, et d'exécution de ladite étape de calcul en fonction d'une valeur supposée de m et des valeurs mesurées réelles de HR et TT.

4. Procédé selon l'une quelconque des revendications précédentes, caractérisé par la définition de l'instant d'arrivée de l'impulsion comme étant le moment où la pression sanguine mesurée qui augmente atteint une amplitude qui est à mi-distance entre la pression de crête de l'impulsion et la pression de creux qui précède l'arrivée de l'impulsion.

5. Appareil d'affichage d'une valeur ou d'un indice de pression sanguine dans un corps vivant humain ou animal, qui comporte un dispositif de détection de l'émission des ondes électrocardiographiques R, un dispositif de détection de l'arrivée des impulsions sanguines correspondantes dans un vaisseau sanguin choisi, et un dispositif de calcul i) d'un temps TT qui s'écoule entre le moment détecté de l'émission de chaque onde R et le moment détecté de l'arrivée de l'impulsion correspondante de pression, et ii) d'un rythme cardiaque HR qui est un nombre détecté d'ondes R apparaissant dans une unité de temps déterminée, l'appareil étant caractérisé par un dispositif (24) de calcul qui est destiné a) à former le produit de HR et TT, b) à diviser ce produit (TT.HR) par un rythme cardiaque $HR_c$ mesuré lorsque le sujet est au repos afin qu'un paramètre $TT.HA/HR_c$ soit obtenu, c) à calculer une valeur ou un indice de la pression sanguine diastolique à l'aide de la fonction suivante F1.

$$DBP = m.TT.(HR/HR_c) + I \qquad F1$$

dans laquelle :

m est la pente de la droite correspondant à la meilleure représentation de la variation de la pression sanguine diastolique absolue (portée en ordonnées) en fonction de $TT.HR/HR_c$ (porté en abscisses),

$HR_c$ est un rythme cardiaque au repos,

I est l'intersection de la droite avec l'axe des ordonnées, et

m, $HR_c$ et I sont des constantes numériques déterminées pendant la procédure d'étalonnage, et d) à afficher la valeur ou l'indice de pression sanguine diastolique.

6. Appareil selon la revendication 5, caractérisé en ce que le dispositif de calcul (24) est programmé afin qu'il transmette un indice de pression diastolique d'après une valeur supposée de m et une valeur réelle de $HR_c$ qui est propre au corps considéré.

7. Appareil selon la revendication 5 ou 6, caractérisé en ce que le dispositif de calcul est programmé afin qu'il calcule les valeurs réelles de m et I qui sont propres au corps considéré, à partir de la fonction F1 et des valeurs réelles, obtenues de manière classique, de la pression diastolique qui sont introduites.

8. Appareil selon l'une quelconque des revendications 5 à 7, caractérisé en ce que le dispositif de calcul est programmé afin qu'il détermine la fin du temps écoulé TT comme étant le moment où la pression sanguine croissante qui marque l'arrivée d'une impulsion atteint une valeur (p + q)/2 qui est à mi-distance (13) entre la pression permanente q qui précède l'impulsion (10) et la pression de crête p qui est atteinte pendant l'impulsion.

9. Appareil selon l'une quelconque des revendications 5 à 8, caractérisé en ce qu'il est destiné à détecter des impulsions de pression dans des vaisseaux sanguins du bout (70) d'un doigt d'un corps humain.

10. Appareil selon l'une quelconque des revendications 5 à 9, caractérisé en ce qu'il donne un mode chronométrique qui peut être utilisé avec la fonction de contrôle cardiaque.

**Patentansprüche**

1. Verfahren zur Anzeige von innerhalb eines Körpers von lebenden Menschen oder Tieren herrschenden Blutdruckwerten oder Blutdruckzahlen, bei den die Aussendung der elektrokardiographischen R-Zacken erfaßt wird, die Ankunft der darauf folgenden Blutdruckimpulse an einem ausgewählten Blutgefäß erfaßt wird und bei dem i) eine Zeit TT berechnet wird, die zwischen dem erfaßten Zeitpunkt der Aussendung der jeweiligen R-Zacke und dem erfaßten Zeitpunkt der Ankunft des darauf folgenden Druckimpulses verstrichen ist, sowie ii) eine Herzfrequenz HR berechnet wird, die eine erfaßte Zahl von in einer gegebenen Zeiteinheit vorkommenden R-Zacken ist, dadurch gekennzeichnet, daß es folgende Schritte aufweist:

a) Bildung des Produktes aus HR und TT;

b) Teilen dieses Produktes (TT.HR) durch die Herzfrequenz $HR_c$, die gemessen wird, wenn der Patient in Ruhe ist, um den Parameter $TT.HR / HR_c$ zu erhalten,

c) Berechnung eines diastolischen Blutdruckwertes oder Blutdruckzahl unter Benutzung der nachstehenden Funktion F1:

$$DBP = m \cdot TT \cdot \frac{HR}{HR_c} + I \qquad (F1)$$

wobei

m die Steigung der Geraden ist, die die Veränderung des absoluten diastolischen Blutdruckes (Ordinate), aufgetragen über

$$TT \cdot \frac{HR}{HR_c}$$

(Abszisse), am besten annähert,
$HR_c$ eine Herzfrequenz in Ruhe ist;
I der Schnittpunkt der Geraden mit der Ordinate ist;
und m, $HR_c$ und I numerische Konstanten sind, die während des Kalibrierungsprozesses bestimmt wurden, sowie
d) Anzeigen des diastolischen Blutdruckwertes oder der Blutdruckzahl.

2. Verfahren nach Anspruch 1, dadurch gekennzeichnet, daß ein Kalibrierungsprozeß für den jeweiligen Körper, für den der diastolische Blutdruckwert anzuzeigen ist, durchgeführt wird, wobei dieser Prozeß die konventionelle Messung der absoluten diastolischen Blutdrücke, der Herzfrequenzen und der verstrichenen Zeitintervalle TT zur Bestimmung der Konstanten m, $HR_c$ und I für den in Frage stehenden Körper umfaßt.

3. Verfahren nach Anspruch 1, dadurch gekennzeichnet, daß $HR_c$ und ein korrespondierender Wert von TT für den jeweiligen Körper eingeführt werden, für den der diastolische Blutdruck anzuzeigen ist, und daß der Berechnungsschritt auf der Basis eines angenommenen Wertes von m und der aktuellen gemessenen Werten von $HR_c$ und TT durchgeführt wird.

4. Verfahren nach einem der vorstehenden Ansprüche, dadurch gekennzeichnet, daß der Zeitpunkt der Ankunft des Impulses als der Zeitpunkt definiert wird, in dem der gemessene ansteigende Blutdruck eine Höhe erreicht, die mittig zwischen dem Scheitelwert des Druckimpulses und dem Wert in dem Drucktal liegt, das der Ankunft des Impulses vorausgeht.

5. Gerät zur Anzeige von innerhalb eines Körpers von lebenden Menschen oder Tieren herrschenden Blutdruckwerten oder Blutdruckzahlen, mit Mitteln für die Erfassung der Aussendung der elektrokardiographischen R-Zacken, Mitteln zur Erfassung der Ankunft der darauf folgenden Blutdruckimpulse an einem ausgewähltem Blutgefäß und mit Mitteln zur Berechnung i) einer Zeit TT, die von dem erfaßten Zeitpunkt der Aussendung jeder R-Zacke bis zu dem erfaßten Zeitpunkt der Ankunft des darauf folgenden Druckimpulses verstrichen ist, und ii) einer Herzfrequenz HR, die eine erfaßte Zahl von in einer gegebenen Zeiteinheit vorkommenden R-Zacken ist, dadurch gekennzeichnet, daß Rechenmittel (24) vorgesehen sind, um:
a) das Produkt aus HR und TT zu bilden,
b) dieses Produkt (TT.HR) durch die Herzfrequenz $HR_c$ zu teilen, die am in Ruhe befindlichen Patienten gemessen wurde, um einen Parameter TT.HR / $HR_c$ zu erhalten,
c) einen diastolischen Blutdruckwert oder eine Blutdruckzahl unter Benutzung der untenstehenden Funktion F1 zu berechnen:

$$DBP = m \cdot TT \cdot \frac{HR}{HR_c} + I \qquad (F1)$$

wobei
m die Steigung der Geraden ist, die die Veränderung des absoluten diastolischen Blutdruckes (Ordinate), aufgetragen über

$$TT \cdot \frac{HR}{HR_c}$$

(Abszisse) am besten annähert,

$HR_c$ eine Herzfrequenz bei Ruhe ist;

I der Schnittpunkt der Geraden mit der Ordinate ist;

und m, $HR_c$ und I numerische Konstanten sind, die während des Kalibrierungsprozesse bestimmt wurden, sowie

d) den diastolischen Blutdruckwert oder die Blutdruckzahl anzuzeigen.

6. Gerät nach Anspruch 5, dadurch gekennzeichnet, daß das Rechenmittel (24) programmiert ist, einen diastolischen Blutdruckwert oder eine Blutdruckzahl abzugeben, der oder die auf einem angenommenen Wert von m und einem tatsächlichen Wert von $HR_c$ beruht, der für den in Frage stehenden Körper spezifisch ist.

7. Gerät nach Ansruch 5 oder 6, dadurch gekennzeichnet, daß das Rechenmittel so programmiert ist, daß es die für den in Frage stehenden Körper spezifischen tatsächlichen Werte von m und I, auf der Basis von F1 sowie von eingegebenen, auf herkömmliche Weise erhaltenen tatsächlichen Werten des diastolischen Drucks berechnet.

8. Gerät nach einem der Ansprüche 5 bis 7, dadurch gekennzeichnet, daß das Rechenmittel derart programmiert ist, daß es das Ende der verstrichenen Zeit TT als den Zeitpunkt bestimmt, zu dem der steigende Blutdruck, der die Ankunft des Impulses markiert, einen Wert von

$$\frac{p + q}{2}$$

erreicht, der in der Mitte zwischen dem statischen Druck q, der dem Impuls (10) vorangeht und dem Scheiteldruck p liegt, der während des Impulses erreicht wird.

9. Gerät nach einem der Ansprüche 5 bis 8, dadurch gekennzeichnet, daß es zur Erfassung von Druckimpulsen in Blutgefäßen in der Fingerspitze (70) eines Menschen eingerichtet ist.

10. Gerät nach einem der Ansprüche 5 bis 9, dadurch gekennzeichnet, daß es einen Zeitmeß - Modus aufweist, der in Verbindung mit seiner Herzüberwachungsfunktion benutzt werden kann.

Fig.I.

Fig.2.

Fig.3.

Fig.4.

EP 0 181 067 B1

FIG.5.

FIG.6.